# EUROPEAN PATENT APPLICATION

(11) **EP 4 597 101 A1**
(43) Date of publication of application: **06.08.2025**
(21) Application number: 23885412.9
(22) Date of filing: 28.09.2023
(51) Int. Cl.: G01N 33/49

(54) **MEASUREMENT METHOD, MEASUREMENT SYSTEM AND TEST REAGENT KIT**

(30) Priority: 04.11.2022 EP 22306660
(71) Applicant: HORIBA, Ltd., Minami-ku Kyoto-shi Kyoto 601-8510 (JP); Horiba ABX SAS, 34790 Grabels (FR)
(72) Inventor: MANIO Mark Christian Carizon, Kyoto-shi, Kyoto 601-8510 (JP); NISHIMURA TANIKAWA Jun Ivan, Kyoto-shi, Kyoto 601-8510 (JP); IGUSHI Tatsuo, Kyoto-shi, Kyoto 601-8510 (JP); DONNARUMMA Dario, 34184 Montpellier, Cedex 4 (FR)
(74) Representative: Isarpatent
(86) International application number: PCT/JP2023/035443
(87) International publication number: WO 2024/095648

(57) **Abstract**

A method, just requiring simple pretreatment, of concentration measurement for reticulocytes or nucleated erythrocytes includes a staining step, a removal step, and a measurement step. In the staining step, reticulocytes or nucleated erythrocytes among red blood cells contained in a blood specimen are stained. In the removal step, the blood specimen is mixed with a surfactant to extract hemoglobin from the red blood cells. In the measurement step, using the blood specimen having undergone the staining step and the removal step, the concentration of the reticulocytes or nucleated erythrocytes contained in the blood specimen is measured.

## Description

### Technical Field

The present invention relates to a method and a system for measuring the concentration of reticulocytes or nucleated erythrocytes in a blood specimen, and relates also to a test reagent kit for use in concentration measurement for reticulocytes or nucleated erythrocytes.

### Background

Reticulocytes are juvenile red blood cells that have just entered peripheral blood from the bone marrow, and mature into red blood cells in one day. Erythroblasts in the bone marrow have a nucleus, and as they mature, they become denucleated to lose the nucleus. In incompletely mature reticulocytes, RNA (ribonucleic acid) as a remnant of the nucleus components is observed in the form of a granular substance with a reticular pattern. An increase in reticulocytes indicates an increase in the production of red blood cells in the bone marrow, and is observed, for example, in treatment of anemia by administration of a iron preparation. On the other hand, in aplastic anemia, due to a drop in hematopoiesis, reticulocytes cease to increase. Thus, measuring the number (concentration) of reticulocytes is useful in diagnosing, and in monitoring a therapeutic course of, a various types of anemia.

For measurement of reticulocyte concentration, for example, according to Patent Document 1 identified below, reticulocytes are stained with a staining reagent (K₃EDTA, NMB (new methylene blue), NaCl) and then, using an erythrocyte hemolytic reagent (KSCN, 1N H₂SO₄), hemoglobin is eluted from red blood cells. Reducing the hemoglobin count allows improved identification of reticulocytes and permits the counting of reticulocytes by flow cytometry.

### Citation List

### Patent Literature

Patent Document 1: JP-A-8-510330

### Summary

### Technical Problem

According to Patent Document 1, the osmotic pressure of the erythrocyte hemolytic liquid is set to 75 to 110 milliosmols, and preferably 82 to 105 milliosmols. The reasons are as follows. For one thing, with too low an osmotic pressure, the erythrocyte hemolytic reagent damages erythrocyte cells, leading to diminished reliability of the reticulocyte count. For another, with an insufficient osmotic pressure, erythrocyte cells do not elute hemoglobin that makes distinction of reticulocytes difficult.

Moreover, according to Patent Document 1, the pH of the erythrocyte hemolytic reagent is set in a range of about 1.0 to 3.0, and preferably in a range of about 1.0 to 2.0. This is because it is believed that an acidic erythrocyte hemolytic reagent dissolves hemoglobin and thereby makes the removal of hemoglobin from erythrocyte cells easy.

With a technique, like the one disclosed in Patent Document 1, that uses an erythrocyte hemolytic reagent to elute hemoglobin from red blood cells, in order to achieve appropriate elution of hemoglobin, it is necessary to adjust the osmotic pressure and the pH of the erythrocyte hemolytic reagent appropriately as described above. This requires complicated pretreatment prior to concentration measurement for reticulocytes.

Incidentally, the need for complicated pretreatment prior to concentration measurement can arise also in concentration measurement for nucleated erythrocytes. Since nucleated erythrocytes contain nucleic acids such as RNA, the same method can be used in concentration measurement with nucleated erythrocytes as in concentration measurement with reticulocytes. Since nucleated erythrocytes, like reticulocytes, are immature red blood cells, detection of nucleated erythrocytes in peripheral blood of an adult is believed to indicate a rise in hematopoiesis for some cause.

Against the background discussed above, an object of the present invention is to provide a method, a system, and a test reagent kit that permit concentration measurement for reticulocytes or nucleated erythrocytes with simple pretreatment.

### Solution to Problem

According to one aspect of the present invention, a method of measuring the concentration of reticulocytes or nucleated erythrocytes contained in a blood specimen includes: a staining step of staining, using a staining reagent, the reticulocytes or nucleated erythrocytes among the red blood cells contained in the blood specimen; a removal step of removing hemoglobin from the red blood cells by mixing the blood specimen with a surfactant; and a measurement step of measuring the concentration of the reticulocytes or nucleated erythrocytes contained in the blood specimen using the blood specimen having undergone the staining step and the removal step.

According to another aspect of the present invention, a test reagent kit includes: a staining reagent for staining reticulocytes or nucleated erythrocytes among the red blood cells contained in a blood specimen; and a surfactant for extracting hemoglobin from the red blood cells.

According to yet another aspect of the present invention, a system for measuring the concentration of reticulocytes or nucleated erythrocytes contained in a blood specimen includes: a storage that stores a staining reagent for staining the reticulocytes or nucleated erythrocytes and a surfactant for mixing with the blood specimen to extract hemoglobin from red blood cells contained in the blood specimen; a flow cell into which the blood specimen, the staining reagent, and the surfactant are introduced; a blood cell volume size measurement unit that measures variation of the volume of blood cells in the blood specimen introduced into the flow cell; an optical measurement unit that measures the light transmissivity of the blood cells in the blood specimen introduced into the flow cell; and a concentration determination unit that determines the concentration of the reticulocytes or nucleated erythrocytes contained in the blood specimen based on the results of the measurement by the blood cell volume size measurement unit and the optical measurement unit.

### Advantageous Effects of Invention

According to the present invention, it is possible to perform concentration measurement for reticulocytes or nucleated erythrocytes with simple pretreatment.

### Brief Description of Drawings

FIG. 1 is a perspective view showing an exterior configuration of a blood analysis apparatus employed in a measurement system according to one embodiment of the present invention;
FIG. 2 is an illustrative diagram schematically showing an internal configuration of the blood analysis apparatus;
FIG. 3 is a block diagram schematically showing another configuration of the measurement system;
FIG. 4 is a flow chart of a procedure for measuring the concentration of reticulocytes by a first measurement method;
FIG. 5 is an illustrative diagram schematically showing a model of a red blood cell before and after ghosting using a surfactant;
FIG. 6 is an illustrative diagram showing an outline of a structure of an LMNE chamber;
FIG. 7 is a plot of the results that an electrical resistance measurement unit and an optical measurement unit yielded for given blood cells with no surfactant added to a blood specimen;
FIG. 8 is a plot of the results that the electrical resistance measurement unit and the optical measurement unit yielded for the same blood cells with a surfactant added to the blood specimen;
FIG. 9 is a flow chart showing a procedure for measuring the concentration of reticulocytes by a second measurement method;
FIG. 10 is a flow chart showing a procedure for measuring the concentration of reticulocytes by a third measurement method;
FIG. 11 is a flow chart showing a procedure for measuring the concentration of reticulocytes by a fourth measurement method;
FIG. 12 is a perspective view showing one example of a reticulocyte test reagent kit;
FIG. 13 is a perspective view showing another example of a reticulocyte test reagent kit; and
FIG. 14 is an illustrative diagram showing an outline of a configuration of an optical apparatus for detecting reticulocytes using a fluorescent dye.

### Description of Embodiments

Illustrative embodiments of the present invention will be described below with reference to the accompanying drawings.

### [1. Outline of a Blood Analysis Apparatus]

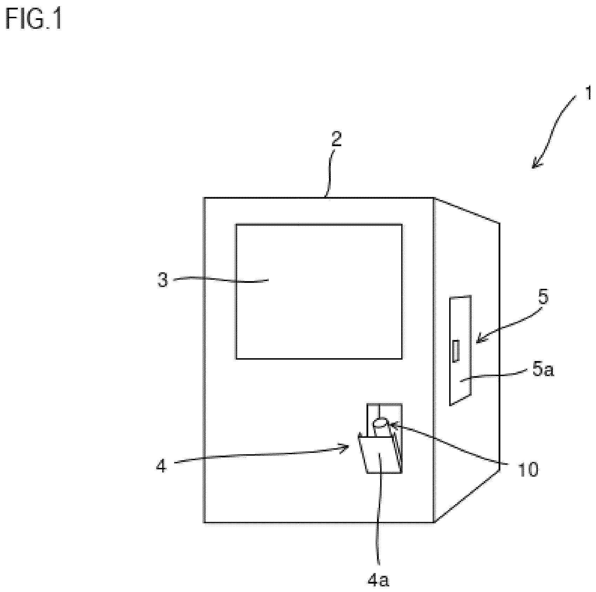

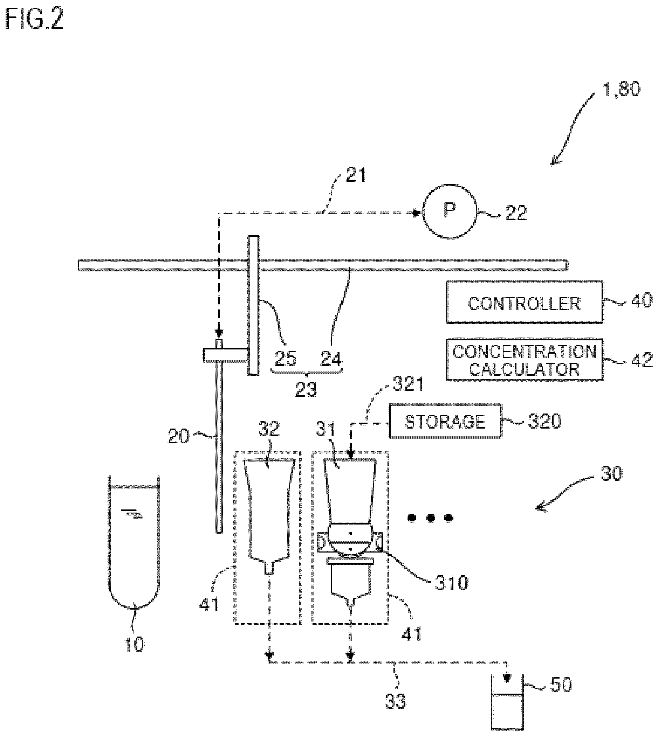

FIG. 1 is a perspective view showing an exterior configuration of a blood analysis apparatus 1 employed in a measurement system 80 (see.Fig.2) according to an embodiment of the present invention. The blood analysis apparatus 1 includes a display 3 in an upper part of the front face of an apparatus body 2. The display 3 is configured as, for example, a liquid crystal display device fitted with a touch panel. The display 3 displays blood analysis results and the like, and also accepts input of various kinds of information from an operator (e.g., a medical professional).

In a lower part of the apparatus body 2, a specimen container loader 4 is provided. A specimen container 10 can be loaded in the apparatus body 2 by opening a cover 4a of the specimen container loader 4, putting there the specimen container 10 with a blood specimen in it, and then closing the cover 4a.

In a side part of the apparatus body 2, a reagent container loader 5 is provided. Opening a door 5a of the reagent container loader 5 permits the apparatus body 2 to be loaded with a container that contains reagents (e.g., a reagent for immunoassay, a reagent for staining reticulocytes), a surfactant, a diluent, and the like to be used in blood analysis. Such a container may be one that contains, for example, a staining reagent, a surfactant, and a diluent together as will be mentioned later, or a plurality of such containers may be used one for each reagent to be used. The reagents mentioned above may be stored in a storage 320 that leads to a chamber 31 (see FIG. 2) via a duct 321.

### [2. Internal Configuration of the Blood Analysis Apparatus]

FIG. 2 is an illustrative diagram schematically showing an internal configuration of the blood analysis apparatus 1. The blood analysis apparatus 1 includes a needle 20, a blood analyzer 30, and a controller 40. The controller 40 is configured, for example, as a data processor (computer) generally known as a CPU (central processing unit), and controls the operation of individual blocks in the blood analysis apparatus 1.

The needle 20 sucks and ejects the blood specimen contained in the specimen container 10. As necessary, the needle 20 pierces a lid of the specimen container 10 to suck and eject the blood specimen. A base end part of the needle 20 is connected to a metering syringe 22 via a pipe 21, indicated by a broken line, and a solenoid valve device (not shown). The controller 40 drives the metering syringe 22 and the solenoid valve device so as to make the needle 20 suck and eject the blood specimen.

A probe unit 23 permits the needle 20 to move in the horizontal and vertical directions. The probe unit 23 includes a horizontal movement mechanism 24 and a vertical movement mechanism 25. The horizontal and vertical movement mechanisms 24 and 25 each include, for example, an endless belt, a driving roller and a driven roller for turning the endless belt, and the like. As the controller 40 drives the respective driving rollers in the horizontal and vertical movement mechanisms 24 and 25, the endless belt in the horizontal movement mechanism 24 runs in the horizontal direction, and the endless belt in the vertical movement mechanism 25 runs in the vertical direction. This permits the needle 20 held on the vertical movement mechanism 25 to move in the horizontal and vertical directions.

The horizontal and vertical movement mechanisms 24 and 25 may each be configured with a feed-screw mechanism. A feed-screw mechanism makes a feed screw rotate with a stepping motor so that an engaged part that is engaged with the feed screw moves in the horizontal or vertical direction. Thus, coupling the vertical movement mechanism 25 to the engaged part of the horizontal movement mechanism 24 and holding the needle 20 on the engaged part of the vertical movement mechanism 25 permits the needle 20 to move in the horizontal and vertical directions.

The blood analyzer 30 analyzes blood as by blood cell counting and immunoassay. The blood analyzer 30 includes a chamber 31 (container) into which the needle 20 ejects the blood specimen it has sucked from the specimen container 10. A chamber 31 for blood cell counting is provided with a counting device 310. According to the type of blood cells to be counted, the counting device 310 can employ different measurement methods including those based on impedance, flow cytometry, and focused flow impedance. The controller 40 can process measurement data acquired by the counting device 310 to yield a frequency distribution or the like. The counting device 310 may be provided outside the chamber 31 and connected to the chamber 31.

The chamber 31 may comprise separate chambers for different types of blood cells to be counted. The separate chambers 31 include, for example, a BASO chamber, an LMNE chamber, an RBC chamber, and a WBC chamber. The BASO chamber is for counting basophils included in leukocytes. The LMNE chamber is for counting lymphocytes, monocytes, neutrophils, and eosinophils included in leukocytes. "LMNE" consists of the initials of lymphocyte, monocyte, neutrophil, and eosinophil. The RBC chamber is for counting red blood cells. The WBC chamber is for counting white blood cells and analyzing HGB (hemoglobin).

The chamber 31 may further comprise a CRP measurement chamber. The CRP measurement chamber is a chamber configured to optically measure a CRP level by latex agglutination. "CRP" is short for C-reactive protein. The CRP measurement chamber includes, for CRP measurement, a light emitter and a light detector on a bottom wall surface of the chamber, and is configured to be able to stir, as necessary, the liquid contained inside.

Near the CRP measurement chamber, a plurality of reagent containers (not shown) are provided. The reagent containers contain a hemolytic reagent, a diluent, an antihuman CRP sensitized latex immunoreagent, and the like respectively. In CRP measurement, with appropriate timing, the reagents from those reagent containers are ejected into the CRP measurement chamber via a duct (not shown).

The blood analyzer 30 includes a cleaning container 32. The cleaning container 32 is for cleaning the needle 20. By cleaning the needle 20 in the cleaning container 32, it is possible to eliminate unwanted matter such as blood and reagents attached to the needle 20, and to dispose of surplus blood specimen in the needle 20.

To bottom-end parts of the chamber 31 and the cleaning container 32 mentioned above, a waste pipe 33 indicated by broken lines are connected. As a discharger (not shown) is driven, the waste liquid inside the chamber 31 and the cleaning container 32 is delivered to a waste container 50 via a solenoid valve device (not shown). The discharger is configured with a discharge syringe or a discharge pump.

When a specimen container 10 containing a blood specimen is loaded in the specimen container loader 4 (see FIG. 1) in the apparatus body 2, the controller 40 drives the probe unit 23 to move the needle 20 in the horizontal and vertical directions. In this way, the needle 20 can be moved into and out of the specimen container 10 and the chamber 31. In addition, the controller 40 can drive the metering syringe 22 to make the needle 20 suck and eject the blood specimen. Through such operation, blood analysis (such as blood cell counting and immunoassay) is performed in the blood analyzer 30.

The blood analyzer 30 further includes a storage 320. The storage 320 stores a staining reagent, a surfactant, and a diluent as will be described later. In the embodiment, the storage 320 stores the just-mentioned staining reagent, surfactant, and diluent together in a mixture; instead, separate storage spaces may be provided to store them separately. The storage 320 is connected to the chamber 31 (e.g., an LMNE chamber) via the duct 321. Thus, the staining reagent, surfactant, and diluent stored in the storage 320 can be fed to the chamber 31 (e.g., the LMNE chamber) via the duct 321.

In the embodiment, two LMNE chambers are provided. The intention is to clearly separate from each other a chamber used for the counting of lymphocytes, monocytes, neutrophils, and eosinophils and a chamber used for the measurement of reticulocyte concentration, which will be described later. A configuration is however also possible where one LMNE chamber doubles as one for the counting of lymphocytes etc. and one for the measurement of reticulocyte concentration.

The blood analysis apparatus 1 further includes a temperature adjuster 41. The temperature adjuster 41 is configured with, for example, a heater, and heats the chamber 31 and the cleaning container 32 to a desired temperature (e.g., 37°C). This helps prevent a blood specimen that agglutinates on exposure to cold from agglutinating. While FIG. 2 shows a configuration where the temperature adjuster 41 is provided one for each of the chamber 31 and the cleaning container 32 and these are heated separately, a configuration is also possible where the temperature adjuster 41 is provided so as to enclose the chamber 31 and the cleaning container 32 together and these are heated simultaneously.

The temperature adjuster 41 may be configured with a tank (also called a buffer tank) with a heater wound around it. The tank stores, for example, a diluent. Such a temperature adjuster 41 is connected to, for example, another pipe (not shown) that branches off the pipe 21 via a switch valve (not shown). The diluent heated by the heater returns to the pipe 21 via the just-mentioned pipe and the just-mentioned switch valve, and is ejected into the chamber 31 via the needle 20. At this time, the blood specimen that the needle 20 has previously sucked from the specimen container 10 is ejected, simultaneously with the heated diluent, into the chamber 31. Thus, also in this case, the blood specimen is heated to a desired temperature inside the chamber 31 by being mixed with the heated diluent, and this prevents the blood specimen from agglutinating. It is preferable that the temperature adjuster 41 be so configured as to heat also reagents (e.g., surfactant) other than the diluent to the above-mentioned desired temperature (e.g., 37°C) from the viewpoint of promoting appropriate reactions.

The blood analysis apparatus 1 also includes a concentration calculator 42. The concentration calculator 42 determines the concentration of reticulocytes contained in the blood specimen based on the counting result (e.g., the number of reticulocytes described later) from the counting device 310 in the blood analyzer 30 and the amount of blood specimen used in the counting. Such a concentration calculator 42 may be configured with the same CPU as, or a separate CPU from, the one in the controller 40, or may be configured as a processing circuit dedicated to concentration determination.

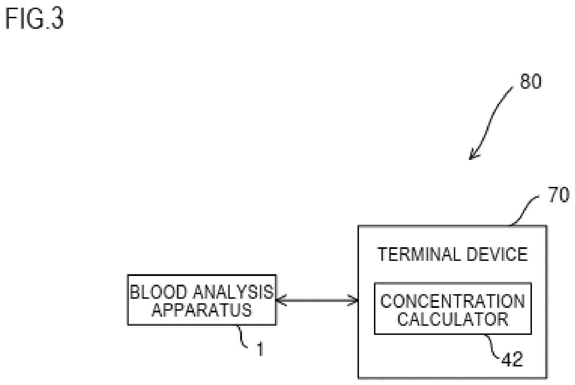

The concentration calculator 42 may be provided outside the blood analysis apparatus 1 and connected to the blood analysis apparatus 1 such that they can communicate with each other. FIG. 3 is a block diagram schematically showing another configuration of the measurement system 80. The measurement system 80 shown there includes a blood analysis apparatus 1 and a terminal device 70. The terminal device 70 is connected to the blood analysis apparatus 1 across a communication network such that they can communicate with each other. The communication network may be wired or wireless.

The terminal device 70 is configured with, for example, a personal computer, and includes, as well as a keyboard and/or any other input device and a display, a concentration calculator 42. The concentration calculator 42 is configured with, for example, the CPU provided in the terminal device 70, and determines the concentration of reticulocytes contained in the blood specimen based on the measurement result (e.g., the number of reticulocytes) output from the counting device 310 in the blood analysis apparatus 1 30 and the amount (volume) of blood specimen entered on the terminal device 70.

### [3. Methods of Measuring the Concentration of Reticulocytes]

### (3-1. First Measurement Method)

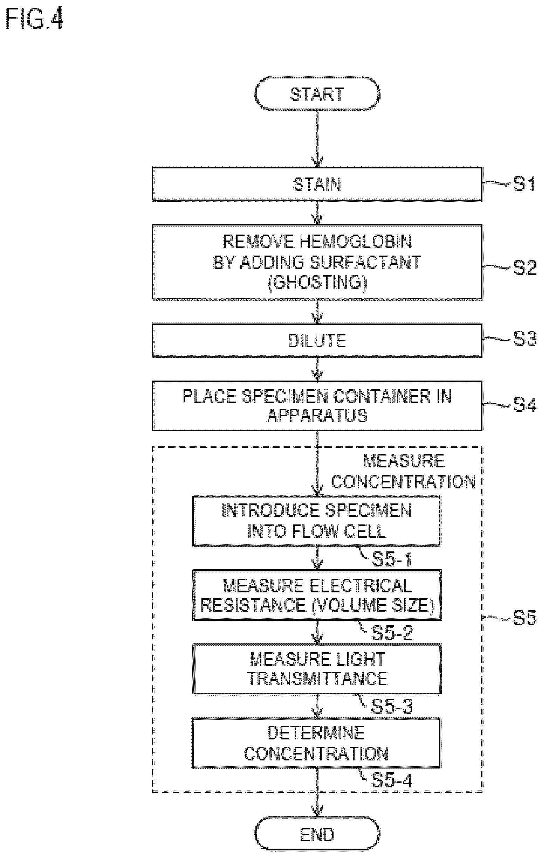

Next, a description will be given of a method of measuring the concentration of reticulocytes on the measurement system 80. FIG. 4 is a flow chart of a procedure for measuring the concentration of reticulocytes by a first measurement method. The first measurement method includes a staining step (S1), a removal step (S2), a dilution step (S3), a loading step (S4), and a measurement step (S5). Of these steps, steps S1 to S3 are performed prior to the measurement step S5 and are called preceding steps. It should be noted that the first measurement method does not use the reagents (staining reagent etc.) stored in the storage 320, and instead uses a blood specimen having reagents added to it outside the apparatus. A detailed description follows.

### (S1: Staining Step)

In the staining step, among the red blood cells contained in the blood specimen collected in the specimen container 10 (see FIG. 1), reticulocytes are stained. The reticulocytes are stained with a staining reagent called NMB (new methylene blue, C₁₈H₂₂ClN₃S). NMB is a non-fluorescent staining reagent with a cationic (+) molecule. Reticulocytes contain RNA (ribonucleic acid)-ribosome complex. Adding NMB to reticulocytes causes the just-mentioned complexes in the reticulocytes to agglutinate into a reticular pattern while involving other subcellular organelles. The RNA in the agglutinated complexes is stained blue. Thus, the reticulocytes are stained.

The staining reagent used in the staining step is not limited to NMB mentioned above. Also usable as the staining reagent is, for example, Azure B (C₁₅H₁₆ClN₃S), a chromophore, or a fluorophore. Among these, NMB, Azure B, and a chromophore are suitable as the staining reagent.

### (S2: Removal Step)

In the removal step, a surfactant is mixed in all or part of the blood specimen stained in S1, and thereby hemoglobin is extracted from red blood cells. A step, like this, of extracting hemoglobin from red blood cells is called ghosting. From the viewpoint of achieving a good balance between the amount of blood specimen and the amount of surfactant, it is preferable that the surfactant be mixed in part of the blood specimen stained in S1.

As the surfactant mentioned above, it is preferable to use a cationic surfactant (positively charged), a zwitterionic surfactant, or a non-ionic surfactant. The reason is as follows. Nucleic acids such as RNA is negatively charged, and NMB is positively charged; if the surfactant is anionic (negatively charged), it may bond to NMB before NMB bonds to RNA, and this may block the staining with NMB.

Usable as a cationic surfactant is, for example, one based on a quaternary ammonium salt (QAS). Surfactants based on a quaternary ammonium salt include, for example, DTAB (dodecyltrimethylammonium bromide) and HTAC (hexadecyltrimethylammonium chloride).

A zwitterionic surfactant is a surfactant that has functional groups of both positive and negative polarities. Usable as a zwitterionic surfactant is, for example, one based on ammonio propane sulfonate. Surfactants based on ammonio propane sulfonate include, for example, DDAPS (N-dodecyl-N N-dimethyl-3-ammonio-1-propanesulfonate).

Non-ionic surfactants include, for example, Tergitol (a registered trademark of Union Carbide Corporation), Triton X-100 ("Triton" is a registered trademark of Union Carbide Corporation), and saccharide fatty acid esters. Saccharide fatty acid esters include, for example, sucrose fatty acid esters (sugar esters) such as DK Ester (a registered trademark of DKS [Daiichi Kogyo Seiyaku] Co. Ltd.) SS.

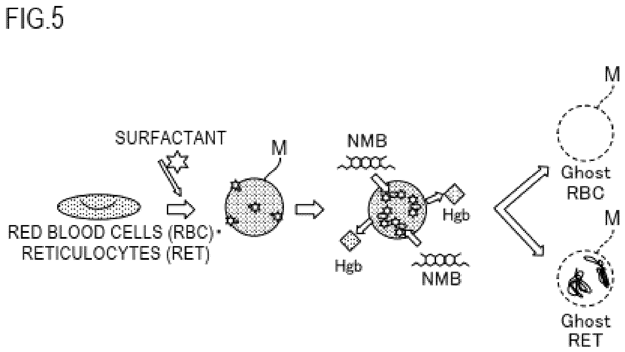

FIG. 5 schematically shows a model of a red blood cell before and after ghosting using a surfactant. The surfactant acts to puncture the membrane M of the red blood cell; as a result, soluble proteins, including hemoglobin (Hgb), and the like ooze out of the red blood cell. The red blood cell in this state, when irradiated with light, absorbs less light with hemoglobin; thus, red blood cells (ghost RBCs) other than reticulocytes appear transparent. Here, the membrane M of the red blood cell has not disappeared and is still present. On the other hand, reticulocytes (ghost RETs), of which the RNA in the above-mentioned agglutinated complexes is stained blue with NMB as described above, appear blue. The surfactant may have any osmotic pressure and any pH value; for example, it can be given an osmotic pressure and a pH value that are close to those of blood.

### (S3: Dilution Step)

In the dilution step, the blood specimen having hemoglobin extracted from red blood cells is diluted with a diluent. Usable as the diluent is, for example, phosphate-buffered saline (PBS).

### (S4: Loading Step)

In the loading step, the specimen container 10 containing the blood specimen after dilution in S3 is placed in the specimen container loader 4 in the blood analysis apparatus 1. Instead of the specimen container 10 being placed in the specimen container loader 4, the blood specimen after dilution may be sucked from the specimen container 10 with a pipette and the sucked blood specimen may be, with the panel (not shown) of the blood analysis apparatus 1 opened, injected directly into the chamber 31 (in particular, the LMNE chamber). This eliminates the need for the loading step for placing the specimen container 10 in the specimen container loader 4.

### (S5: Measurement Step)

In the measurement step, the blood specimen having undergone staining, ghosting, and dilution is subjected to measurement of the concentration of reticulocytes contained in the blood specimen. In the embodiment, the blood analysis apparatus 1 includes, as the chamber 31 in the blood analyzer 30, an LMNE chamber. Reticulocyte concentration measurement can be performed using the LMNE chamber. In particular, in a case where, as in the embodiment, the blood analysis apparatus 1 includes a plurality of LMNE chambers, the concentration of reticulocytes can be measured using any of the LMNE chambers. The above-mentioned blood specimen subjected to the measurement step may be a blood specimen contained in the specimen container 10 placed in the specimen container loader 4 in S4, or a blood specimen injected directly into an LMNE chamber with a pipette.

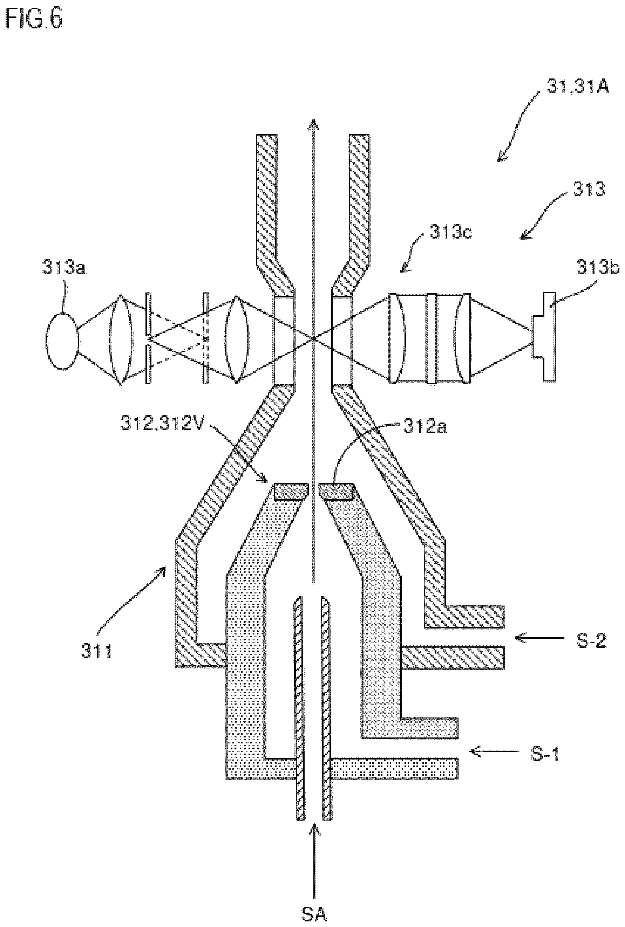

FIG. 6 is an illustrative diagram showing an outline of a structure of an LMNE chamber 31A as the chamber 31. The LMNE chamber 31A includes a flow cell 311, an electrical resistance measurement unit 312, an optical measurement unit 313, and the counting device 310 (see FIG. 2) mentioned earlier.

The blood specimen mixed with the staining reagent (NMB), the surfactant, and the diluent is introduced into the flow cell 311 (S5-1: an introduction step). The introduction of the blood specimen into the flow cell 311 is achieved by the needle 20 (see FIG. 2) sucking the blood specimen from the specimen container 10 and ejecting it into the flow cell 311. The blood specimen introduced into the flow cell 311 will be referred to also as the "sample SA". Instead, in S5-1, the blood specimen may be injected directly into the flow cell 311 with a pipette.

The sample SA in the form of a fine stream surrounded by sheath fluid S-1 passes through an aperture 312a provided in the electrical resistance measurement unit 312. Meanwhile, variation of electrical resistance is sensed as pulses with platinum electrodes (not shown) arranged at opposite ends of the aperture 312a. In this way, the electrical resistance measurement unit 312 measures the size of the cells (blood cells) that pass through the aperture 312a (S5-2: an electrical resistance measurement step).

Subsequently, the sample SA in a state surrounded by sheath fluid S-2 is led to the optical measurement unit 313. The optical measurement unit 313 includes a light source 313a (e.g., a halogen lamp), a detector 313b (a photocell), and an optical system 313c. The optical system 313c is located between the light source 313a and the detector 313b. The optical system 313c includes lenses and optical filters. As the light source 313a emits light toward the sample SA (blood specimen) passing through the flow cell 311, the light transmitted through the blood cells (red blood cells, white blood cells) in the sample SA reaches the detector 313b. The detector 313b thus senses the light transmittance (light transmissivity) of the blood cells (S5-3: an optical measurement step). Based on the results of the measurement by the electrical resistance measurement unit 312 and the optical measurement unit 313, the counting device 310 counts the number of reticulocytes contained in the sample SA (blood specimen). Then, based on the result of the counting of reticulocytes and the amount of blood specimen, the concentration calculator 42 determines the concentration of reticulocytes (S5-4: a concentration determination step). It is here assumed that the amount (volume) of blood specimen is known beforehand.

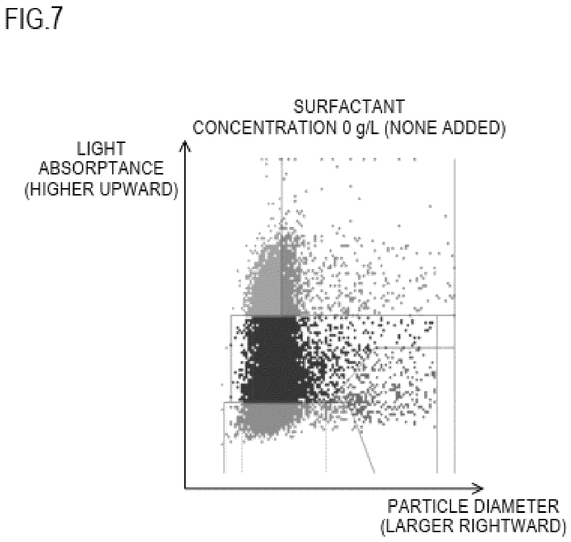

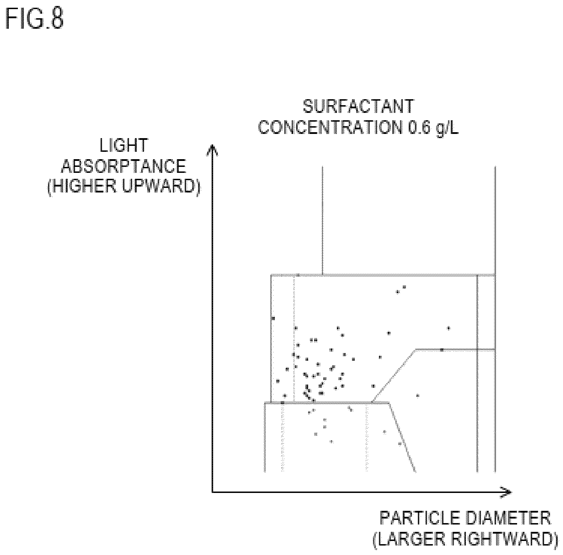

For example, FIG. 7 is a plot of the results that the electrical resistance measurement unit 312 and the optical measurement unit 313 yielded for given blood cells with the concentration of Tergitol as the surfactant at 0 g/L, that is, with no Tergitol added to the blood specimen in S2. By contrast, FIG.8 is a plot of the results that the electrical resistance measurement unit 312 and the optical measurement unit 313 yielded for the same blood cells with the concentration of Tergitol as the surfactant at 0.6 g/L. In these plots, the horizontal axis represents the results of the measurement by the electrical resistance measurement unit 312 (i.e., the particle diameter of blood cells) such that, the farther rightward, the greater the particle diameter; the vertical axis represents the results of the measurement by the optical measurement unit 313 (i.e., the light transmittance of blood cells) such that, the farther upward, the higher the light absorptance (the lower the light transmittance).

By keeping constant the speed of the flow of the sample SA passing through the flow cell 311 and thereby keeping constant the time lag between the timing of electrical resistance measurement and the timing of optical transmittance measurement, it is possible to obtain the results of electrical resistance measurement (i.e., particle diameter) and the results of optical transmittance measurement (i.e., light absorptance) for the same blood cells.

As a consequence of, in the embodiment, the LMNE chamber 31A being used for reticulocyte concentration measurement, in FIGS. 7 and 8, the boundaries among the five types of white blood cells detectable in the LMNE chamber 31A are left to appear on the plots. These boundaries, however, can be ignored here.

With no surfactant added to the blood specimen, hemoglobin is not removed from red blood cells; hence, when the blood specimen containing blood cells of varying particle diameters are irradiated with light, the hemoglobin in the red blood cells absorbs a large amount of light. This causes the optical measurement unit 313 to optically detect not only reticulocytes but also mature red blood cells. Hence a distribution as shown in FIG. 7 that reflects the light absorbance detected for blood cells of varying particle diameters including reticulocytes and mature red blood cells. This makes it difficult for the counting device 310 to accurately count only reticulocytes based on the results of the measurement by the electrical resistance measurement unit 312 and the optical measurement unit 313.

By contrast, with a surfactant added to the blood specimen, hemoglobin is removed from red blood cells, and this helps reduce the light absorbed in red blood cells. The optical measurement unit 313 can then optically detect only reticulocytes. Thus, by counting the points plotted in FIG. 8, that is, the points corresponding to the results of the measurement by the electrical resistance measurement unit 312 and the optical measurement unit 313, the counting device 310 can count only reticulocytes. The concentration calculator 42 can thus accurately determine the concentration of reticulocytes.

Thus, with the first measurement method described above, before the concentration of reticulocytes contained in a blood specimen is measured, hemoglobin is removed from red blood cells using a surfactant. As the surfactant, as described above, one with an osmotic pressure and a pH value close to those of blood can be used. In this way, simply by mixing the surfactant with the stained blood specimen, it is possible to easily remove hemoglobin from the red blood cells. This eliminates the need for pretreatment, as required in a conventional method employing an erythrocyte hemolytic reagent, for appropriately adjusting the osmotic pressure and the pH value of the erythrocyte hemolytic reagent. This helps simplify the pretreatment prior to reticulocyte concentration measurement. It is thus possible to perform reticulocyte concentration measurement with simple pretreatment.

In particular, the surfactant is cationic, zwitterionic, or non-ionic. This helps restrain the surfactant from bonding to NMB (positively charged) before NMB bonds to RNA (negatively charged) contained in reticulocytes. It is thus possible to stain RNA with NMB without fail.

By performing the staining step (S1), the removal step (S2), and the dilution step (S3) in this order (in three steps), it is possible to proceed with pretreatment steadily step by step.

For example, a high concentration of the surfactant may make reticulocyte concentration measurement impossible by breaking the membrane of red blood cells during transfer to the flow cell 311 after removal of hemoglobin from red blood cells. Performing the dilution step (S3) as described above reduces the concentration of the surfactant, and thereby makes a hindrance as mentioned above less likely. In reticulocyte concentration measurement, the dilution step is not indispensable. For example, a low concentration of the surfactant permits reticulocyte concentration measurement to be performed without going through the dilution step (with it skipped). This applies equally to the other measurement methods described below.

The measurement step S5 includes an introduction step (S5-1), an electrical resistance measurement step (S5-2), an optical measurement step (S5-3), and a concentration determination step (S5-4). Thus, semiautomatic operation is possible in which, while S1 through S4 are performed manually, S5 is performed on the blood analysis apparatus 1 (and the terminal device 70).

While the description thus far deals with a method of measuring the concentration of reticulocytes with a blood specimen introduced in the LMNE chamber 31A as the chamber 31, in a case where the blood analysis apparatus 1 includes, other than the LMNE chamber 31A, a chamber dedicated to reticulocyte concentration measurement, the concentration of reticulocytes may be measured with the blood specimen introduced into that dedicated chamber.

While the above description of S5-2 deals with an example where the size of blood cells (in other words, variation of the volume of blood cells) is measured based on variation of the electric resistance observed when blood cells pass through the aperture 312a (see FIG. 6), it is also possible to measure the size of blood cells by an optical technique. For example, blood cells can be passed between a light-emitter and a light-receiver and, based on the amount of light received by the light-receiver, the size of blood cells can be measured. Thus, the electrical resistance measurement step S5-2 can be said to be one example of a blood cell volume size measurement step for measuring variation of the volume of blood cells in the blood specimen passing through the flow cell 311. On the other hand, the electrical resistance measurement unit 312 can be said to be one example of a blood cell volume size measurement unit 312V (see FIG. 6) for measuring the electrical resistance of blood cells in the blood specimen passing through the flow cell 311 and thereby measuring variation of the volume of the blood cells.

### (3-2. Second Measurement Method)

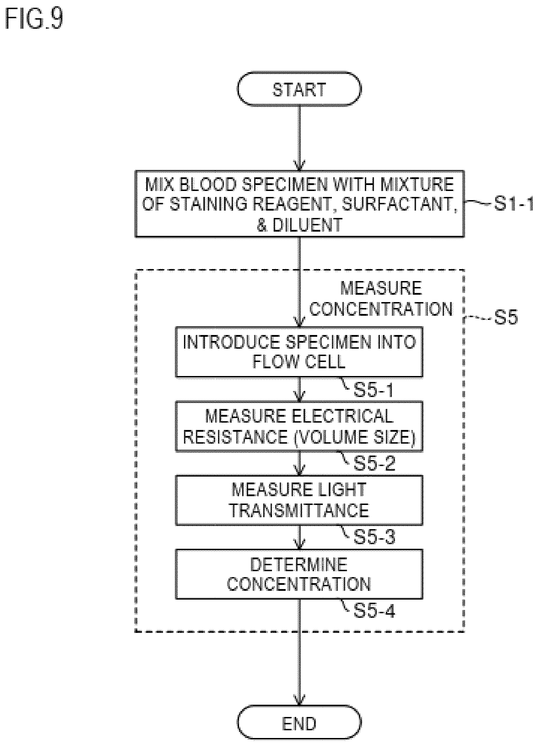

In the first measurement method described above, the three steps from S1 through S3 can be consolidated into a single step. Now, as another example of a method of reticulocyte concentration measurement, a second measurement method will be described. FIG. 9 is a flow chart showing a procedure for measuring the concentration of reticulocytes by the second measurement method.

First, a blood specimen is mixed with a mixture of a staining reagent (NMB), a surfactant, and a diluent simultaneously (S1-1: a mixture adding step). Thus, the staining of reticulocytes contained in the blood specimen, the removal of hemoglobin from red blood cells by the surfactant, and the dilution of the blood specimen take place concurrently. That is, step S1-1 corresponds to a step in which the staining step (S1), the removal step (S2), and the dilution step (S3) described previously are performed concurrently. As in FIG. 3, S1-1 is followed by step S5. In S5-1 in S5, for example, the blood specimen after dilution is sucked from the specimen container 10 with a pipette and the sucked blood specimen is injected directly into an LMNE chamber in the blood analysis apparatus 1; instead, a specimen container 10 is loaded in the specimen container loader 4, and a blood specimen in the specimen container 10 may be sucked with the needle 20 and then ejected into the LMNE chamber.

Performing the staining step, the removal step, and the dilution step concurrently (in a single step) in S1-1 requires only one-time mixing of the blood specimen with chemical agents (such as reagents). This, compared with the first measurement method, which performs pretreatment in three steps, helps further simplify the pretreatment prior to concentration measurement.

### (3-3. Third Measurement Method)

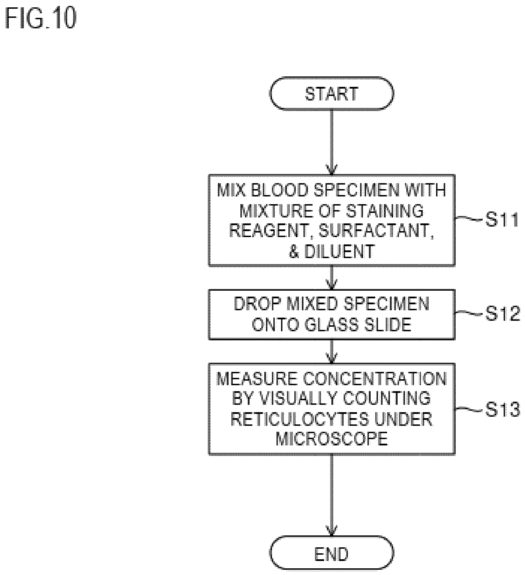

Reticulocyte concentration measurement can also be performed based on the result of visual measurement. Now, a method of measuring concentration by visually counting the number of reticulocytes will be described as a third measurement method. FIG. 10 is a flow chart showing a procedure for measuring the concentration of reticulocytes by the third measurement method.

First, a blood specimen is mixed with a mixture of a staining reagent (NMB), a surfactant, and a diluent simultaneously (S1-1: a mixture adding step). Step S11 is the same as S1-1 in FIG. 9.

Next, a predetermined amount of the blood specimen mixed with the mixture in S1-1 is dropped onto a glass slide (S12: a dropping step). After that, the glass slide is placed on a microscope, and the stained reticulocytes are counted visually. The count of reticulocytes observed in the predetermined amount mentioned above is determined as the concentration of reticulocytes (S13: a concentration determination step).

As in the first measurement method, the pretreatment may be performed in a sequence of three steps (staining step, removal step, and loading step), and then the steps in S12 and the following steps in FIG. 10 may be performed. That is, first, the three steps mentioned above may be performed, and then a predetermined amount of the diluted blood specimen mixed may be dropped onto a glass slide (S12); then the number of stained reticulocytes can be counted visually under a microscope, and then, based on the result of the measurement, the concentration of reticulocytes can be determined.

Regardless of whether the pretreatment is performed in three steps or in a single step, by performing S12 and S13 thereafter, it is possible to measure reticulocytes visually and determine the concentration of reticulocytes.

It is also possible to photograph with a camera the field of view observed under a microscope, then subject the photographed image to image processing, then counting reticulocytes by identifying them based on the shapes of red blood cells eventually obtained through the image processing, and thereby measure the concentration of reticulocytes.

### (3-4. Fourth Measurement Method)

Reticulocyte concentration measurement can be performed full-automatically on the measurement system 80. Now, a method of fully automatic reticulocyte concentration measurement will be described as a fourth measurement method. In the fourth measurement method, the reagents (a mixture of a staining reagent, a surfactant, and a diluent) stored in the storage 320 shown in FIG. 2 is used. A detailed description follows.

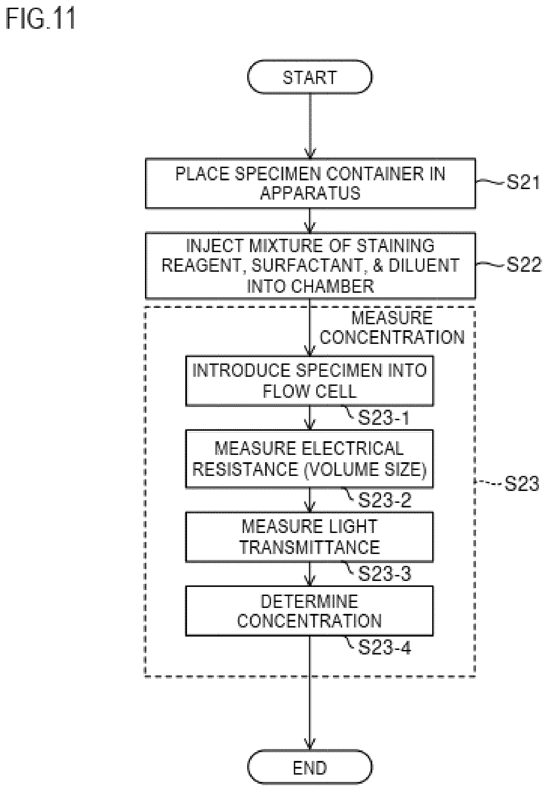

FIG. 11 is a flow chart showing a procedure for measuring the concentration of reticulocytes by the fourth measurement method. The fourth measurement method includes a loading step (S21), a mixture adding step (S22), and a measurement step (S23).

In the loading step S21, the specimen container 10 containing a blood specimen is loaded in the specimen container loader 4 (see FIG. 1) in the blood analysis apparatus 1.

In the mixture adding step S22, a mixture of a staining reagent, a surfactant, and a diluent is fed from the storage 320 via the duct 321 to the chamber 31 (in particular, the LMNE chamber). The mixture adding step S22 corresponds to a step in which S1 (staining step), S2 (removal step), and S3 (dilution step) in FIG. 4 are performed in a single step (concurrently).

The measurement step S23 includes an introduction step (S23-1), an electrical resistance measurement step (S23-2), an optical measurement step (S23-3), and a concentration determination step (S23-4). The measurement step S23 corresponds to the measurement step (S5) in FIG. 4. Accordingly, the introduction step (S23-1), the electrical resistance measurement step (S23-2), the optical measurement step (S23-3), and the concentration determination step (S23-4) included in the measurement step S23 correspond respectively to the introduction step (S5-1), the electrical resistance measurement step (S5-2), the optical measurement step (S5-3), and the concentration determination step (S5-4) shown in FIG. 4. In the measurement step S23, as with the first measurement method, the concentration of reticulocytes contained in the blood specimen is determined.

In the introduction step S23-1, the blood specimen is sucked with the needle 20 from the specimen container 10 loaded in the specimen container loader 4, and is ejected into the chamber 31 (in particular, the LMNE chamber). After the passage of a predetermined time to allow reactions to progress between the blood specimen and the mixture of the staining reagent etc. fed to the chamber 31 via the duct 321 (at least the time required for staining and hemoglobin removal), the reactant inside the chamber 31 moves to the flow cell 311.

As described above, with the fourth measurement method, the staining step, the removal step, and the dilution step are performed on the blood analysis apparatus 1 provided with the flow cell 311 (S22). Thus, the whole procedure from the staining of reticulocytes through the measurement of their concentration can be performed full-automatically on the measurement system 80.

In a configuration where the storage 320 in the blood analysis apparatus 1 stores a staining reagent, a surfactant, and a diluent separately, it is possible to adopt a configuration where the staining reagent, the surfactant, and the diluent are fed from the storage 320 to the chamber 31 one after the other. By so doing it is possible to achieve a concertation determination method that performs the staining step, the removal step, and the dilution step in three steps, with shifted timing, all full-automatically.

### [4. Reticulocyte Test Reagent Kit]

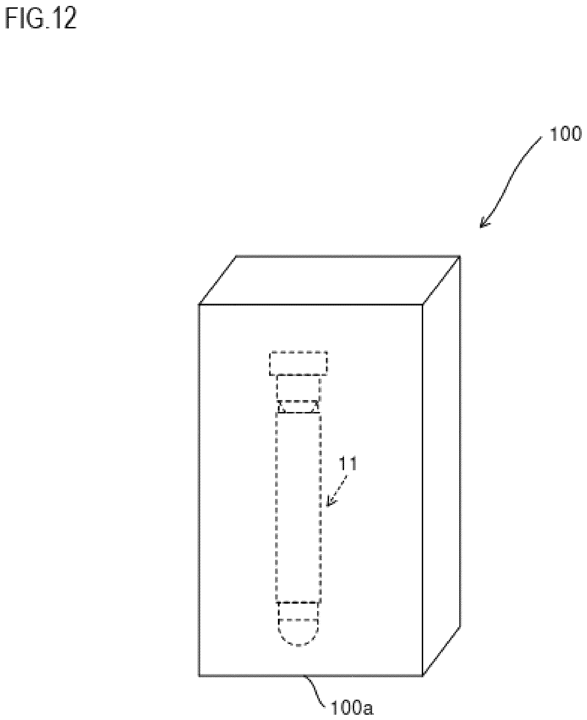

FIG. 12 is a perspective view showing one example of a reticulocyte test reagent kit 100. The staining reagent, surfactant, and diluent mentioned above can be offered to a user (e.g., medical professional) in the form of a reticulocyte test reagent kit 100. In the reticulocyte test reagent kit 100, a container 11 that contains a mixture of a staining reagent, a surfactant, and a diluent is packed in packing material 100a. That is, the reticulocyte test reagent kit 100 includes a staining reagent, a surfactant, and a diluent.

The user takes the container 11 out of the packing material 100a in the reticulocyte test reagent kit 100. The user can then suck the mixture from the container 11 with a pipette or the like and inject it, outside a blood analysis apparatus 1, into a specimen container 10 containing a blood specimen. In this way, the user can implement the second measurement method described above. The user can also drop the mixture sucked with the pipette or the like onto a glass slide or inject it directly into a chamber 31 in the blood analysis apparatus 1. In that case, the user can implement the third and fourth measurement methods described above.

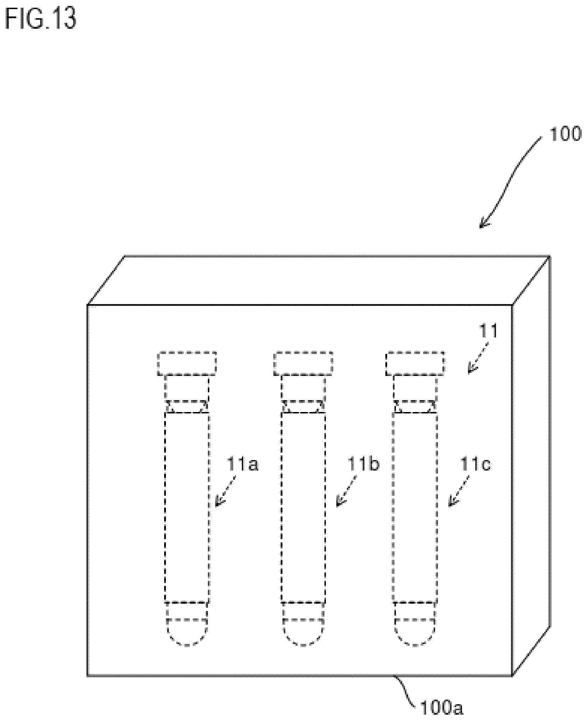

FIG. 13 is a perspective view showing another example of a reticulocyte test reagent kit 100. The reticulocyte test reagent kit 100 may include, as the specimen container 10, a first container 11a containing a staining reagent, a second container 11b containing a surfactant, and a third container 11c containing a diluent.

As shown in FIG. 13, owing to the staining reagent, surfactant, and diluent being contained in separate containers (the first, second, and third containers 11a, 11b, 11c), the user can take those containers individually out of the packing material 100a in the reticulocyte test reagent kit 100 and then inject, outside the blood analysis apparatus 1, the staining reagent, surfactant, and diluent one after the other with a pipette or the like into the specimen container 10 containing a blood specimen. In this way, the user can implement the first measurement method described above.

A reticulocyte test reagent kit 100 can include at least a staining reagent and a surfactant out of a staining reagent, a surfactant, and a diluent. Diluents are in general use as fluids for diluting blood specimens, and a user may have them on hand. Accordingly, if a reticulocyte test reagent kit 100 incudes a staining reagent and a surfactant, it is possible to offer to a user the minimum materials for reticulocyte concentration measurement to enable the user to perform reticulocyte concentration measurement according to the embodiment. In particular, if the reticulocyte test reagent kit 100 also includes a diluent, it is possible to offer to a user all the materials necessary for reticulocyte concentration measurement to enable the user to perform reticulocyte concentration measurement according to the embodiment without fail.

### [5. Reticulocyte Detection Using a Fluorescent Dye]

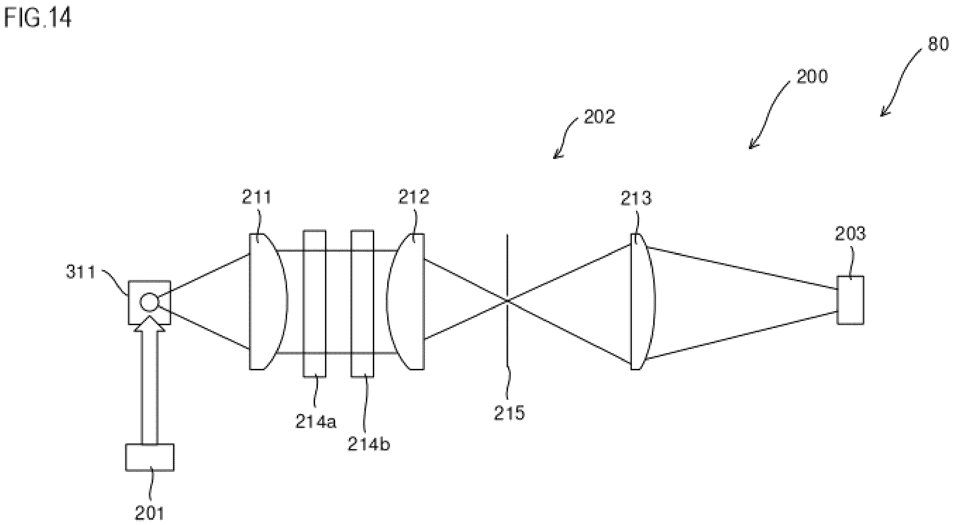

Reticulocytes can be detected also using a fluorescent dye. FIG. 14 is an illustrative diagram showing an outline of a configuration of an optical apparatus 200 for detecting reticulocytes using a fluorescent dye. The optical apparatus 200 can be employed in the measurement system 80 described above. The optical apparatus 200 includes a laser light source 201, a detection optical system 202, and a detector 203. The laser light source 201 emits laser light of a desired wavelength (e.g., 488 nm). The detection optical system 202 is composed of three converging lenses 211, 212, and 213, optical filters 214a and 214b, and a spatial filter 215.

The optical filter 214a (first optical filter) is a filter for cutting light of the same wavelength as the laser light. The optical filter 214b (second optical filter) is a filter for cutting Raman-scattered light (in particular, of the Raman wavelength of water). The optical filters 214a and 214b are arranged between the two converging lenses 211 and 212 closer to the laser light source 201, in the mentioned order from the laser light source 201 side. The spatial filter 215 is arranged between the two condensing lenses 212 and 213 and has an aperture (pinhole). The detector 203 is configured as, for example, a Si-PMT (photomultiplier tube).

After the RNA contained in reticulocytes is stained with a fluorescent dye, a blood specimen is introduced into the flow cell 311. Irradiating the cells (reticulocytes stained with the fluorescent dye) passing through the flow cell 311 with the laser light from the laser light source 201 produces, on one hand, light excited by irradiation with the laser light (i.e., fluorescence) and, on the other hand, elastically scattered light of the same wavelength as the laser light from the cells. The elastically scattered light from the cells is cut by the optical filter 214a in the detection optical system 202 so that only the fluorescence reaches the detector 203. Moreover, irradiating the cells passing through the flow cell 311 with the laser light also causes the laser light to be scattered by the liquid (e.g., water) around the cells to produce Raman-scattered light (inelastically scattered light) with a wavelength longer than that of the laser light. However, the Raman-scattered light so produced is cut by the optical filter 214b in the detection optical system 202. Thus, the detector 203 detects fluorescence under reduced influence of the Raman-scattered light.

An optical system with an inexpensive and simple configuration as achieved by inclusion of an optical filter 214b for cutting Raman-scattered light as described above helps enhance the accuracy of reticulocyte detection. In particular, the Si-PMT employed in the detector 203 has superb sensitivity, and this is advantageous in enhancing the accuracy of reticulocyte detection. By contrast, a configuration where, for example, an APD (avalanche photodiode) is used as the detector 203, because of the small size of the APD, requires an expensive optical system or an optical adjustment mechanism for converging light finely as well as the associated adjustment operation. By contrast, as compared with the APC, the Si-PMT not only excels in sensitivity but provides a larger light-receiving area; this eliminates the need for an expensive optical system or the like as well as the associated adjustment operation, and allows adoption of a configuration employing an inexpensive, low-output laser light source 201.

### [6. Measuring the Concentration of Nucleated Erythrocytes]

The methods and the system 80 for reticulocyte concentration measurement and the reticulocyte test reagent kit 100 for use in reticulocyte concentration measurement described above are usable also in the measurement of the concentration of nucleated erythrocytes. That is, as described earlier, nucleated erythrocytes contain nucleic acids such as RNA, and thus it is possible to measure the concentration of nucleated erythrocytes by going through the steps of staining the Nucleic acids , removing hemoglobin with a surfactant, and the like as in concentration measurement for reticulocytes.

While the present invention has been described by way of embodiments, this is not meant to limit the scope of the present invention, which can thus be implemented with any expansions or modifications made without departure from the spirit of the invention.

### Industrial Applicability

The present invention finds application in measurement of the concentration of reticulocytes or nucleated erythrocytes.

### Reference Signs List

- 1: blood analysis apparatus
- 20: needle
- 42: concentration calculator
- 80: measurement system
- 100: reticulocyte test reagent kit (test reagent kit)
- 201: laser light source (light source)
- 202: detection optical system
- 203: detector
- 214a: optical filter (first optical filter)
- 214b: optical filter (second optical filter)
- 310: counting device
- 311: flow cell
- 312: electrical resistance measurement unit
- 312V: blood cell volume size measurement unit
- 313: optical measurement unit
- 320: storage

## Claims

1. A method of measuring a concentration of reticulocytes or nucleated erythrocytes contained in a blood specimen, the method comprising:
a staining step of staining, using a staining reagent, the reticulocytes or nucleated erythrocytes among red blood cells contained in the blood specimen;
a removal step of removing hemoglobin from the red blood cells by mixing the blood specimen with a surfactant; and
a measurement step of measuring the concentration of the reticulocytes or nucleated erythrocytes contained in the blood specimen using the blood specimen having undergone the staining step and the removal step.

2. The method according to claim 1, further comprising:
a dilution step of diluting the blood specimen.

3. The method according to claim 2, wherein
the staining step, the removal step, and the dilution step are performed in this order.

4. The method according to claim 2, wherein
the staining step, the removal step, and the dilution step are performed concurrently.

5. The method according to any one of claims 1 to 4, wherein
the measurement step includes:
an introduction step of introducing the blood specimen into a flow cell;
a blood cell volume size measurement step of measuring variation of a volume of blood cells in the blood specimen passing through the flow cell;
an optical measurement step of measuring light transmittance of the blood cells in the blood specimen passing through the flow cell; and
a concentration determination step of determining the concentration of the reticulocytes or nucleated erythrocytes contained in the blood specimen based on results of the measurement in the blood cell volume size measurement step and the optical measurement step.

6. The method according to claim 5, wherein
the blood cell volume size measurement step includes:
an electrical resistance measurement step of measuring variation of an electrical resistance of the blood cells in the blood specimen passing through the flow cell, thereby to measure the variation of the volume of the blood cells.

7. The method according to claim 5 or 6, wherein
the staining step and the removal step are performed on a blood analysis apparatus that includes the flow cell.

8. The method according to any one of claims 1 to 4, wherein
the measurement step includes:
dropping a predetermined amount of the blood specimen onto a glass slide,
measuring a number of stained ones of the reticulocytes or nucleated erythrocytes visually under a microscope, and
determining the concentration of the reticulocytes or nucleated erythrocytes based on a result of the measurement.

9. The method according to any one of claims 1 to 8, wherein
the surfactant is cationic, zwitterionic, or non-ionic.

10. A test reagent kit comprising:
a staining reagent for staining reticulocytes or nucleated erythrocytes among red blood cells contained in a blood specimen; and
a surfactant for extracting hemoglobin from the red blood cells.

11. The test reagent kit according to claim 10, further comprising:
a diluent for diluting the blood specimen.

12. A system for measuring a concentration of reticulocytes or nucleated erythrocytes contained in a blood specimen, the system comprising:
a storage configured to store
a staining reagent for staining the reticulocytes or nucleated erythrocytes and
a surfactant for mixing with the blood specimen to extract hemoglobin from red blood cells contained in the blood specimen;
a flow cell configured such that the blood specimen, the staining reagent, and the surfactant are introduced thereinto;
a blood cell volume size measurement unit configured to measure variation of a volume of blood cells in the blood specimen introduced into the flow cell;
an optical measurement unit configured to measure light transmissivity of the blood cells in the blood specimen introduced into the flow cell; and
a concentration determination unit configured to determine the concentration of the reticulocytes or nucleated erythrocytes contained in the blood specimen based on results of the measurement by the blood cell volume size measurement unit and the optical measurement unit.

13. The system according to claim 12, wherein
the blood cell volume size measurement unit includes:
an electrical resistance measurement unit configured to measure variation of an electrical resistance of the blood cells in the blood specimen introduced into the flow cell, thereby to measure the variation of the volume of blood cells.

14. The system according to claim 12 or 13, wherein
the storage further stores a diluent for diluting the blood specimen, and
along with the staining reagent and the surfactant, the diluent is introduced into the flow cell.

15. The system according to any one of claims 12 to 14, further comprising:
a light source configured to emit light toward fluorescently stained cells passing through the flow cell;
a detection optical system; and
a detector configured to detect, via the detection optical system, fluorescence generated by excitation as a result of the cells being irradiated with the light,
wherein
the detection optical system incudes:
a first optical filter configured to cut elastically scattered light that is produced as a result of the cells being irradiated with the light and that has a same wavelength as the light; and
a second optical filter configured to cut Raman-scattered light that is produced as a result of the light being scattered by a liquid around the cells and that has a longer wavelength than the light.
